# EUROPÄISCHE PATENTSCHRIFT

(11) **EP 0 753 354 B1**
(45) Veröffentlichungstag und Bekanntmachung des Hinweises auf die Patenterteilung: **20.03.2002**
(21) Anmeldenummer: 96111001.2
(22) Anmeldetag: 09.07.1996
(51) Int. Cl.: B05B 11/02

(54) **Zerstäuber für fliessfähige Medien, insbesondere für den Austrag in nur einem Hub**
Spraying device for fluids, especially for dispensing in a single stroke
Pulvérisateur pour fluides, en partculier à course unique

(30) Priorität: 14.07.1995 DE 19525734
(43) Veröffentlichungstag der Anmeldung: 15.01.1997
(73) Patentinhaber: ING. ERICH PFEIFFER GMBH, 78315 Radolfzell (DE)
(72) Erfinder: Fuchs, Karl-Heinz, D-78315 Radolfzell (DE); Merk, Hans, D-78343 Gaienhofen (DE)
(74) Vertreter: Patentanwälte Ruff, Wilhelm, Beier, Dauster & Partner

(56) Entgegenhaltungen:
- EP-A- 0 486 894
- EP-A- 0 546 607
- EP-A- 0 580 897
- WO-A-93/00172
- FR-A- 2 667 050
- US-A- 3 187 749
- US-A- 3 739 779

## Beschreibung

Die Erfindung betrifft einen Zerstäuber für fließfähige Medien gemäß dem Oberbegriff des Anspruchs 1. Eine bekannte Austragvorrichtung ist in der DE-A- 40 21 263 beschrieben. Dabei ist in einem Kolbenstopfen eine von dem Betätigungsstößel durchstoßbare Membran vorgesehen, die den Verschluß bildet.

Es sind ferner aus den DE-A- 36 31 341 und 40 05 528 der Anmelderin Kugelventile bekannt, die das Auslaßventil bilden und durch den im Inneren des Medienspeichers bei Betätigung aufgebauten Flüssigkeitsdruck betätigt werden.

Die EP 0 486 864 A1 beschreibt einen Zerstäuber gemäß dem Oberbegriff des Anspruchs 1, bei dem in einem Kolben ein Schieberventil vorgesehen ist, das bei der Betätigung von dem Betätigungsstößel innerhalb des Kolbens axial verschoben wird, bis eine Nut an der Innenwand der Kolbenbohrung mit einem Querkanal in diesem Ventilkörper in Verbindung kommt. Hier handelt es sich um ein Schieberventil innerhalb eines Kolbens. Es benötigt einen mit einer winkligen Bohrung versehenen Ventilkörper sowie eine spezielle Ausarbeitung von Längsnuten 36 in der Kolbenbohrung.

Bei der FR 2 667 050 A1 ist eine Kugel vorgesehen, die die Füllöffnung 71 nach der Füllung des Zerstäubers verschließt. Auch bei der WO 93/00172 dient eine Kugel zum Verschluss der Füllöffnung.

Die US-Patentschriften 3 187 749 und 3 739 779 zeigen kugelförmige Verschlusselemente in Injektionsspritzen. Die Kugeln werden von den Injektionsnadeln ohne wesentlichen Gegendruck herausgestoßen.

Die EP 058 897 A1 weist als Auslassventil eine wulstförmige Ringdichtung 11 auf, die durch Stauchung eines elastischen Abschnittes das Auslassventil öffnet.

Ferner ist in der EP-0 311 863 A der Anmelderin beschrieben, daß ein Pumpenzylinder und ein nach Art einer Schnappverriegelung ausgebildeter federnder Anschlag so zusammenwirken, daß vor dem Austrag eines Teilhubes ein bestimmter Betätigungsdruck von dem Bedienenden aufgebracht werden muß, so daß erst nach Überwindung dieses Druckpunktes der Austrag der Flüssigkeit mit einem Mindestdruck erfolgt. Diese Ausbildung stellt sicher, daß bei Zerstäubung eines Mediums der Druck vom ersten Moment an zur Zerstäubung ausreicht. Dies ist besonders wichtig für Austragvorrichtungen, die ihren gesamten Austrag in nur einem Hub bewirken und die zur Ausgabe von Medikamenten bestimmt sind, die bezüglich der Dosierung, Kontaminierung, Konservierung etc. bis zum wirklichen Gebrauch hermetisch abgeschlossen und somit steril sind. Bei diesen meist sehr kostspieligen Medikamenten muß sichergestellt sein, daß nicht durch eine zu schwache Betätigung der Inhalt z.B. unzerstäubt verlorengeht.

### Aufgabe und Lösung

Aufgabe der Erfindung ist es, einen Zerstäuber der eingangs erwähnten Art zu schaffen, der eine einfache und zuverlässige Ausgabe von Medien aus einem vor der Benutzung sicher verschlossenen Medienspeicher ermöglichen.

Diese Aufgabe wird erfindungsgemäß durch den Anspruch 1 gelöst.

Während bei der eingangs beschriebenen bekannten Ausführung der den Medienspeicher verschließende Kolbenstößel von einem nadelartigen Betätigungsstößel durchtrennt wird, wird bei der Erfindung ein festgelegtes Element zerstörungsfrei in den Behälter hineingedrückt. Dies könnte eine Kugel sein, die z.B. im Durchgangskanal eines Kolbenstopfens eingepreßt ist. Es wird dadurch vermieden, daß beim Durchstoßen des Kolbenstößels Partikel abreißen, die den Austragkanal zusetzen könnten. Ferner wird vermieden, daß durch seitliches Einreißen des Kolbenstößelmaterials sich Nebenkanäle bilden, aus denen die Flüssigkeit unter Umgehung des Austragkanales austreten kann.

Es ist ferner dafür gesorgt, daß das Verschlußelement so festgelegt ist, daß es erst nach Überwindung einer vorgegebenen Druckkraft des Betätigungsstößels eindrückbar ist.

Dadurch wird der Aufbau einer ausreichenden Betätigungskraft sichergestellt, bevor durch Öffnen des Verschlusses der Austrag beginnt.

Die Festlegung des Verschlußelementes kann formschlüssig oder rein kraftschlüssig erfolgen. Es könnte also im Durchgang eines Kolbenstopfens eine umlaufende Ausnehmung vorgesehen sein, in der die das Verschlußelement bildende Kugel liegt. Sie kann sich aber beim Einpressen diese Erweiterung des Innenkanals im aus elastischem Material bestehenden Kolbenstopfen auch selbst bilden. Ferner ist es auch möglich, daß das Verschlußelement selbst elastisch ist. Es könnte sogar unmittelbar am Medienspeicher festgelegt sein, d.h. ohne Zwischenschaltung eines Kolbenstopfens. In diesem Falle wird dann der Betätigungsstößel den Austrag nach Art eines Verdrängerkolbens bewirken.

Normalerweise wird aber mit einem aus elastischem Material, wie Gummi, weichelastischem Kunststoff oder dgl. bestehenden Kolbenstopfen gearbeitet, der von einem zusammen mit dem Betätigungsstößel bewegten Anschlag nach Öffnen des Verschlusses in den Medienspeicher hineingedrückt wird und so den Austragkolben bildet. Diese Anschlagfläche bildet auch eine zusätzliche Abdichtung des Austragkanals im Betätigungsstößel. Die Hauptabdichtung sollte jedoch vorzugsweise durch die Außenfläche des Betätigungsstößels gebildet sein, die in der Öffnung des Kolbenstößels bzw. des Medienspeichers abdichtet.

Diese und weitere Merkmale gehen außer aus den Ansprüchen auch aus der Beschreibung und den Zeichnungen hervor, wobei die einzelnen Merkmale jeweils für sich allein oder zu mehreren in Form von Unterkombinationen bei einer Ausführungsform der Erfindung und auf anderen Gebieten verwirklicht sein und vorteilhafte sowie für sich schutzfähige Ausführungen darstellen können, für die hier Schutz beansprucht wird. Die Unterteilung der Anmeldung in einzelne Abschnitte sowie Zwischen-Überschriften beschränken die unter diesen gemachten Aussagen nicht in ihrer Allgemeingültigkeit.

### Kurzbeschreibung der Zeichnung

Ausführungsbeispiele der Erfindung sind in den Zeichnungen dargestellt und wird im Folgenden näher erläutert. In den Zeichnungen zeigen:
- Fig. 1: einen Längsschnitt durch eine Austragvorrichtung und
- Fig. 2: ein Detail einer Variante.

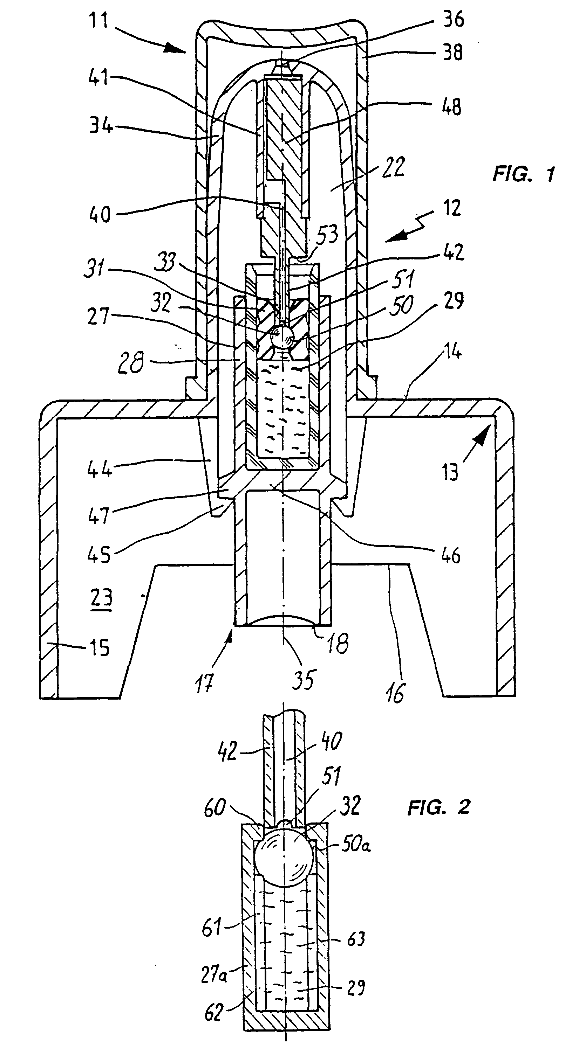

### Beschreibung des Ausführungsbeispiels nach Fig. 1

Die in Fig. 1 dargestellte Austragvorrichtung 11 weist einen Grundkörper 12 auf, der zweiteilig ausgebildet ist. Er enthält einen Betätigungsabschnitt 13, das eine epaulettenartige Form mit zwei Betätigungsschultern 14 und einem daran anschließenden Mantel 15 hat. Der Betätigungsabschnitt ist flach oder flachoval und hat seine größte Erstreckung in der Zeichenebene, während es quer dazu geringere Abmessungen hat. Der Mantel ist an den in Fig. 1 rechten und linken Seiten länger und hat an seinen beiden etwa parallel zur Zeichenebene verlaufenden Seiten einen Ausschnitt 16.

Der Grundkörper 12 umfaßt ferner einen Stutzenabschnitt 34, der nach Art einer oben kugelig geschlossenen Hülse zwischen den beiden Schultern 14 hochragt. Er enthält an seiner äußersten Stelle eine Sprühdüse 36. Im unbenutzten Zustand wird dieser Stutzenabschnitt 34 von einer Schutzhülse 38 überdeckt.

Der Innenraum 22 des Stutzenabschnitts 34 steht mit dem Raum 23 im Mantel in Verbindung. In diesem zusammenhängenden Innenraum 22, 23 ist ein Medienspeicher 27 aufgenommen, der aus einem zylindrischen, einseitig offenen Glasbehälter besteht. Er ist in eine entsprechende Aufnahme 28 einer Betätigungshülse 47 eingesteckt, die, wie (bis auf den Medienspeicher) alle übrigen Teile der Vorrichtung aus Kunststoff besteht. Diese Aufnahme erstreckt sich hülsenförmig von einem Zwischenboden 46 hinweg, der im Bereich eines seitlichen Führungsflansches 47 ausgebildet ist. Zur Betätigungsseite hin erstreckt sich ebenfalls ein hülsenartiger Vorsprung, der an seinem Ende eine ringförmige Betätigungsfläche 18 bildet.

Die Betätigungshülse 17 ist entlang der Mittelachse 35 der Vorrichtung verschiebbar zwischen Haltelaschen 44 angeordnet, die elastisch sind und an ihren Enden widerhakenartige Rastvorsprünge 45 tragen, die hinter den Führungsflansch 47 schnappen und somit nach Einschub der Betätigungshülse von unten her diese gegen Herausfallen sichern. An dieser Stelle können auch bei der Betätigung zerstörbare Materialbrücken zur Bildung einer Originalitätssicherung vorgesehen sein.

Der Medienspeicher enthält das auszugebende Medium 29, beispielsweise ein durch die Haut aufnehmbares Medikament. Er ist durch einen weichelastischen, dichtend eingepreßten Kolbenstopfen 31 verschlossen, der eine innere Öffnung 33 aufweist, in die als Verschlußelement 32 eine Kugel eingepreßt ist. Die Mündungen der Öffnung 33 können trichterförmig erweitert sein.

Wie in Fig. 1 dargestellt ist, liegt diese Kugel in dem Bereich der Öffnung 33, der dem Medium zugewandt ist. Sie ist so groß, daß sie das Material des Kolbenstopfens, das relativ weichelastisch ist, verdrängt und sich dadurch eine Ausnehmung 50 schafft. Dadurch erhöht sie auch den Dichtdruck, den die Außenflächen des Kolbenstopfens 31 auf die Innenwandung des den Pumpenzylinder bildenden Medienspeichers ausüben.

In die Öffnung 33 des Kolbenstopfens 31 greift ein Betätigungsstößel 42 ein, der als ein Rohr ausgebildet ist, das unten weitgehend gerade abgeschnitten, jedoch vorzugsweise mit Querausnehmungen 51 in seiner Mündungsfläche ausgebildet ist, um von der Kugel 32 nicht verschlossen zu werden. Der Außenumfang des Betätigungsstößels 42 paßt dichtend in die Öffnung 33 des Kolbenstopfens 31 hinein. Seine Länge ist beim Ausführungsbeispiel so lang wie der Kolbenstopfen. An ihn schließt sich eine als Anschlagfläche dienende Betätigungsschulter 53 an, die an einem Stößelträger 48 vorgesehen ist. Er ist in eine nach unten stehende Aufnahmehülse 41 des Grundkörpers 13 eingepreßt und bildet mit seiner oberen, der Düse 36 zugewandten Stirnfläche die die Zerstäubung bewirkenden Drallnuten der Düse. Der Betätigungsstößel und der damit einstückige Stößelträger 48 hat einen durchgehenden Austragkanal 40, der im Bereich des Betätigungsstößels durch einen zentralen Innenkanal des rohrstutzenartigen Betätigungsstößels und im oberen Teil durch eine äußere Flüssigkeitsführungsnut gebildet ist.

### Funktion der Ausführungsform nach Fig. 1

Der mit dem auszugebenden Medium 29 gefüllte Medienspeicher 27 und mittels Kolbenstopfen 31 und Verschlußelement 32 verschlossen wird in die Betätigungshülse 17 eingesteckt und von unten her zentral zwischen die Haltelaschen 44 gesteckt, bis er in der dargestellten Position einrastet. Dabei wird bereits der Betätigungsstößel 42 bis kurz vor seine Betätigungsposition gebracht. Wenn nun die Austragvorrichtung betätigt wird, so wird sie durch zwei auf den gegenüberliegenden Betätigungsschultern 14 liegende Finger gehalten, während mit dem Daumen auf die Betätigungsfläche 18 gedrückt wird. Dadurch wird die Betätigungshülse 17 nach oben geschoben, und der Betätigungsstößel 31 trifft auf die Kugel 32, die ihm einen Gegendruck entgegensetzt, der einerseits aus der Einpreßkraft in dem Kolbenstopfen 31 und andererseits aus dem Gegendruck des meist inkompressiblen Mediums 29 herrührt. Dieser wird dadurch überwunden, daß der Betätigungsstößel 42 die Kugel weiter ins Innere des Medienspeichers hineindrückt, wobei sich entsprechend der Kolbenstopfen 31 etwas nach oben bewegen kann oder eine Verformung erfährt, um für die verdrängte Flüssigkeit Raum zu machen.

Wenn die Kugel 32 aus der durchgehenden Bohrung 33 des Kolbenstopfens heraus und in den Medienspeicher hineingedrückt ist, dann ist die zentrale Austragöffnung 40 frei, und das Medium kann sich über diese zur Düse 36 bewegen und dort zerstäubt austreten. Dies geschieht erst, nachdem ein ausreichender Betätigungsdruck aufgebaut ist. Kurz nachdem der Betätigungsstößel die Kugel ganz hineingedrückt hat, kommen die Anschlagflächen 53 an der Oberseite des Kolbenstopfens 31 zur Anlage und drücken nun den Kolbenstopfen unter Austrag des Mediums in den Medienspeicher hinein.

Falls es auf einen vollständigen Austrag des Medium ankommt, könnte der Boden des Medienspeichers und/oder des Kolbenstopfens 31 so ausgenommen sein, daß dort die Kugel in einer passenden Vertiefung zum Liegen kommt.

### Beschreibung der Ausführungsform nach Fig. 2

In Fig. 2 ist bei im übrigen gleichem Aufbau wie gemäß Fig. 1 lediglich der Medienspeicher 27a, das Verschlußelement 32 und der Stößel 42 dargestellt. Es ist zu erkennen, daß dort auf einen Kolbenstopfen verzichtet wurde und das kugelförmige, in diesem Falle selbst elastische Verschlußelement 32 allein den Verschluß des Medienspeichers bildet. Dieser hat an seiner Oberseite einen die Öffnung verengenden Flansch 60, während eine sich daran anschließende Ausnehmung 50a zum Medienspeicher hin durch einzelne rippenartige Vorsprünge 61 begrenzt ist. In dieser seitlichen Ausnehmung 50a dichtet das Verschlußelement 32 ab und wird durch die Vorsprünge 61 daran gehindert, nach innen zu fallen. Diese Vorsprünge setzen sich als Längsrippen über den Medienspeicher fort. Sie können so ausgebildet sein, daß die zwischen ihnen liegenden Fördernuten 62 nur einen relativ kleinen Querschnitt haben.

### Funktion der Ausführungsform nach Fig. 2

Beim Druck des Betätigungsstößels 42 auf das Verschlußelement 32 wird dieses so verformt, daß es über die Vorsprünge 61 hinweg in den zwischen den Vorsprüngen gebildeten Innenraum 63 gedrückt wird. Dadurch kann das Medium über die Fördernuten 62 an dem Dichtelement 32 vorbei und in den Austragkanal 40 strömen, ggf. durch die Querausnehmungen 51. Die Außenfläche des Betätigungsstößels 42 dichtet an der Innenseite des Flansches 60 ab. Der Betätigungsstößel 42 bildet dabei selbst einen Verdrängerkolben.

## Patentansprüche

1. Zerstäuber für fließfähige Medien (29) mit einem Grundkörper (13) für die Aufnahme eines Medienspeichers (27) mit einer Schubkolbenpumpe, insbesondere für den Austrag in nur einem Hub, und einer Sprühdüse mit einem von einer Betätigungskraft in den eine Pumpenkammer bildenden Medienspeicher (27) einführbaren und dabei einen Verschluß des Medienspeichers (27) öffnenden Betätigungsstößel (42), wobei der Verschluß ein an dem Medienspeicher (27) in einem Durchgangskanal (33) festgelegtes, zerstörungsfrei von diesem lösbares und durch den Betätigungsstößel (42) in den Medienspeicher (27) eindrückbares Verschlußelement (32) ist, **dadurch gekennzeichnet, dass** das Verschlusselement (32) eine Kugel ist, die in den Durchgangskanal (33) derart eingepresst ist, daß sie erst nach Überwindung einer vorgegebenen, für den Betätigungsdruck ausreichenden Druckkraft des Betätigungsstößels (42) eindrückbar ist .

2. Zerstäuber nach Anspruch 1, **dadurch gekennzeichnet, daß** der Durchgangskanal (33) in einem Kolbenstopfen (31) vorgesehen ist.

3. Zerstäuber nach Anspruch 1 oder 2, **dadurch gekennzeichnet, daß** das Verschlußelement (32) selbst elastisch ist.

4. Zerstäuber nach einem der vorhergehenden Ansprüche, **dadurch gekennzeichnet, daß** das Verschlußelement (32) an dem Medienspeicher (27a) festgelegt ist.

5. Zerstäuber nach einem der vorhergehenden Ansprüche, **dadurch gekennzeichnet, daß** der Betätigungsstößel (42) ein Rohrstutzen ist.

6. Zerstäuber nach einem der vorhergehenden Ansprüche, **dadurch gekennzeichnet, daß** mit dem Betätigungsstößel (42) eine an dem Kolbenstopfen (31) nach Öffnung des Verschlusses angreifende und diesen zum Austrag bewegende Anschlagfläche (53) verbunden ist.

7. Zerstäuber nach einem der vorhergehenden Ansprüche, **dadurch gekennzeichnet, daß** der Betätigungsstößel (42) eine äußere, mit dem Medienspeicher (27) bzw. ggf. einem darin vorgesehenen Kolbenstopfen (31) zusammenwirkende Dichtfläche aufweist.

## Claims

1. An atomizer for fluid media (29) comprising a main body (13) for accommodating a media reservoir (27) and including a plunger pump, more particularly for single-stroke only discharge, together with a spray nozzle including an actuator (42) introducable by an actuating force into said media reservoir (27) forming a pump chamber and thereby opening a closure of said media reservoir (27), said closure being a closure element (32) defined by said media reservoir (27) in a thru-passage (33), releasable therefrom non-destructively and capable of being urged into said media reservoir (27) by said actuator (42), wherein said closure element (32) is a ball urged into said thru-passage (33) such that it cannot be urged thereinto until a predetermined pressure force of said actuator (42) sufficient for the actuating pressure is overcome.

2. The atomizer as set forth in claim 1, wherein said thru-passage (33) is provided in a plunger plug (31).

3. The atomizer as set forth in claim 1 or 2, wherein said closure element (32) itself is elastic.

4. The atomizer as set forth in any of the preceding claims, wherein said closure element (32) is defined at said media reservoir (27a).

5. The atomizer as set forth in any of the preceding claims, wherein said actuator (42) is a port.

6. The atomizer as set forth in any of the preceding claims, wherein a stop surface (53) is connected to said actuator (42), said stop surface engaging said plunger plug (31) following opening of the closure and moving the latter for discharge.

7. The atomizer as set forth in any of the preceding claims, wherein said actuator (42) comprises an outer sealing surface cooperating with said media reservoir (27) and, if required, with a plunger plug (31) provided therein.

## Revendications

1. Pulvérisateur pour des substances (29) coulantes avec un corps de base (13) pour le logement d' un réservoir à substances (27) avec une pompe à piston mouvant, notamment pour la distribution par une seule course, et avec un gicleur de pulvérisation avec un coulisseau d' actionnement (42), pouvant être introduit par une force d' actionnement dans le réservoir à substances (27), qui forme une chambre de pompe, et ainsi ouvrant un moyen de fermeture du réservoir à substances (27), le moyen de fermeture étant un élément de fermeture (32) fixé auprès du réservoir à substances (27) dans un canal de passage (33), pouvant être détaché de celui-ci sans destruction et pouvant être enfoncé dans le réservoir à substances (27) au moyen du coulisseau d' actionnement (42), **caractérisé en ce que** l'élément de fermeture (32) est une sphère, qui est forcée dans le canal de passage (33) de telle manière qu' elle peut être enfoncée seulement après avoir surmonté une force de pression prédéterminée du coulisseau d' actionnement (42), suffisante pour la pression d' actionnement.

2. Pulvérisateur d' après la revendication 1, **caractérisé en ce que** le canal de passage (33) est prévu dans un bouchon-piston (31).

3. Pulvérisateur d' après la revendication 1 ou 2, **caractérisé en ce que** l' élément de fermeture (32) est lui-même élastique.

4. Pulvérisateur d' après une des revendications précédentes, **caractérisé en ce que** l' élément de fermeture (32) est fixé auprès du réservoir à substances (27a).

5. Pulvérisateur d' après une des revendications précédentes, **caractérisé en ce que** le coulisseau d' actionnement (42) est une tubulure.

6. Pulvérisateur d' après une des revendications précédentes, **caractérisé en ce qu'** une surface d' arrêt (53), laquelle est en prise avec le bouchon-piston (31) après l' ouverture du moyen de fermeture et le pousse à l' action de distribution, est raccordée au coulisseau d' actionnement (42).

7. Pulvérisateur d' après une des revendications précédentes, **caractérisé en ce que** le coulisseau d' actionnement (42) présente une surface d' étanchéité extérieure, agissant ensemble avec le réservoir à substances (27) ou encore éventuellement avec un bouchon-piston (31) prévu à l' intérieur de celui-ci.
